# FASCICULE DE BREVET EUROPEEN

(11) **EP 2 675 823 B1**
(45) Date de publication et mention de la délivrance du brevet: **08.04.2015**
(21) Numéro de dépôt: 12705265.2
(22) Date de dépôt: 15.02.2012
(51) Int. Cl.: C07K 1/00, C07K 1/02

(54) **PROCÉDÉ DE PRÉPARATION DE PEPTIDES PAR ASSEMBLAGE DE MULTIPLES FRAGMENTS PEPTIDIQUES**
VERFAHREN ZUR HERSTELLUNG VON PEPTIDEN DURCH MULTIFRAGMENTKONDENSATION
METHOD FOR PREPARING PEPTIDES BY ASSEMBLING MULTIPLE PEPTIDE FRAGMENTS

(30) Priorité: 16.02.2011 FR 1151279
(43) Date de publication de la demande: 25.12.2013
(73) Titulaire: Centre National de la Recherche Scientifique, 75794 Paris Cedex 16 (FR); Université de Lille 1 Sciences et Technologies, 59655 Villeneuve d'Ascq Cedex (FR)
(72) Inventeur: MELNYK, Oleg, F-59112 Annoeullin (FR); OLLIVIER, Nathalie, F-59100 Roubaix (FR); MHIDIA, Reda, F-59370 Mons en Baroeul (FR); DHEUR, Julien, F-59480 La Bassee (FR)
(74) Mandataire: Hirsch & Associés
(86) Numéro de dépôt international: PCT/EP2012/052548
(87) Numéro de publication internationale: WO 2012/110536

(56) Documents cités:
- NATHALIE OLLIVIER ET AL: "Bis(2-sulfanylethyl)amino Native Peptide Ligation", ORGANIC LETTERS, vol. 12, no. 22, 19 novembre 2010 (2010-11-19), pages 5238-5241, XP055000245, ISSN: 1523-7060, DOI: 10.1021/ol102273u cité dans la demande
- CAMARERO J A ET AL: "UNIT18.4: Native chemical ligation of polypeptides.", CURRENT PROTOCOLS IN PROTEIN SCIENCE, no. Suppl.15, mai 2001 (2001-05), pages 18.4.1-18.4.21, XP002640389, EDITORIAL BOARD, JOHN E. COLIGAN ... [ET AL.] ISSN: 1934-3663

## Description

### DOMAINE DE L'INVENTION

La présente invention concerne un procédé de préparation de peptides ou de polypeptides par assemblage de multiples fragments peptidiques, utilisant la ligation native de polypeptides. L'invention concerne également la préparation d'intermédiaires utilisés dans ce procédé et lesdits intermédiaires préparés par le procédé.

### ARRIERE-PLAN TECHNOLOGIQUE

La synthèse de polypeptides par des méthodes conventionnelles en phase solide, acide aminé par acide aminé, est limitée par des rendements faibles lorsque les polypeptides synthétisés sont de grande taille. Afin de surmonter cette limitation, il est connu d'assembler deux polypeptides par ligation chimique afin de produire un polypeptide plus long.

La synthèse totale de polypeptides est de plus en plus utile pour la préparation de protéines de structures bien définies. Les méthodes de ligation chimique apportent une réponse à ce besoin, cependant elles s'avèrent limitées dans leur emploi et leur application industrielle.

De manière générale, dans ces méthodes, on souhaite que le lien entre les polypeptides assemblés par ligation soit natif, c'est-à-dire corresponde à la structure naturelle des polypeptides.

La principale méthode de ligation native existant à ce jour est celle de Kent et Dawson, décrite par exemple dans les demandes internationales WO 96/34878 et WO 98/28434. Cette méthode est fondée sur une réaction chimiosélective entre un peptide thioester (en C terminal) et un cystéinyl-peptide. Néanmoins, le principal inconvénient de cette méthode est la fabrication des peptides thioesters qui nécessite des procédés chimiques complexes. Notamment, selon un mode d'assemblage décrit par S. Kent (Kinetically Controled Ligation (KCL)), il est nécessaire de synthétiser les fragments A-SAr, H-Cys-B-SAlk, et H-Cys-C. Cependant des fragments de type A-SAr sont difficiles à produire et s'avèrent être sensibles à l'hydrolyse. De plus, ce procédé ne permet pas d'aller au-delà de l'assemblage de 3 fragments.

Une méthode alternative est la ligation dite de Staudinger, décrite dans les demandes internationales WO 01/68565 et WO 01/87920. Celle-ci comprend la réaction d'un phosphinothioester avec un azide et l'hydrolyse des réactifs combinés pour former une liaison amide. Cependant cette méthode est difficilement applicable à l'échelle industrielle.

Une autre méthode, décrite dans la demande internationale WO 2007/037812, repose sur la réaction d'un α-cétoacide avec une alkoxyamine dans une réaction de condensation décarboxylative. Toutefois, les cétoacides sont des molécules qui sont difficiles à fabriquer et à incorporer dans des peptides. Aussi, cette troisième méthode est difficilement applicable dans les laboratoires de synthèse peptidique ne disposant pas des moyens de réaliser des synthèses organiques complexes.

La publication de D. Bang, B.L. Pentelute and S.B.H. Kent, Angew. Chem. Int. Ed. 2006, 45, 3985-3988 propose une voie de synthèse faisant intervenir des peptide-(thiophénylesters) avec des Cys-peptide-thioesters, et la publication de W. Hou et coll., Org Lett., (2010), 22 décembre 2010, propose la formation de peptides thioesters pour la synthèse de protéines. Cependant ces méthodes ne peuvent pas empêcher la compétition entre les réactions des différents thioesters, conduisant inévitablement à des mélanges pouvant être difficilement séparables, donc affectant la pureté du produit final obtenu, et à des pertes inévitables de rendement.

Enfin, la publication de O. Melnyk et coil., Org. Lett., 12(22), 5238-41 (2010) décrit la ligation native de peptides au moyen de fragments peptide-bis(sulfanyléthyl)amino. Cependant cette méthode n'a jamais été utilisée, à ce jour, pour la synthèse de peptides à multiples fragments.

La transposition à l'échelle industrielle de procédés permettant de mettre en oeuvre des synthèses peptidiques par synthèse totale est un besoin qui requiert de trouver des procédés simples, peu coûteux, produisant des produits de qualité de pureté élevée et raisonnables en matière d'hygiène industrielle.

Pour les raisons énoncées ci-dessus, il est devenu indispensable de trouver un procédé de synthèse totale, convergente et industrialisable, permettant de synthétiser un enchaînement peptidique de la longueur et de la nature souhaitée. Particulièrement un procédé mettena en jeu un assemblage du N-terminal vers le C-terminal, qui offre des qualités de simplicité de réalisation et de pureté des peptides ou polypeptides obtenus.

### DESCRIPTION DE L'INVENTION

Il a été trouvé, ce qui fait l'objet de la présente invention, que l'assemblage de multiples fragments peptidiques dans une méthode one-pot mettant en jeu des méthodes simples comme la formation de peptides-thioesters et la ligation native, pouvait conduire à un procédé de synthèse totale, convergente, industrialisable et répondant aux critères de pureté requis.

Selon l'invention, le procédé d'assemblage fait intervenir la synthèse de peptide-thioesters, leur réaction de condensation sur un peptide (ou polypeptide) portant une fonction bis(2-sulfanyléthyl)amino à l'état de disulfure cyclique, répondant à la formule générale : 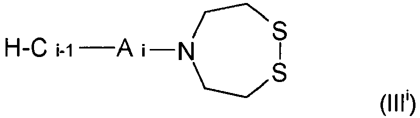 dans laquelle
Aᵢ représente un fragment peptidique dont le C-terminal porte un groupement bis(2-sulfanyléthyl)amino cyclique 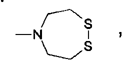 ci-après appelé SEAoff, et
Cᵢ₋₁ représente un résidu d'acide aminé portant une fonction thiol,
ainsi que la ligation chimique native en milieu réducteur, au niveau du C-terminal, des polypeptides obtenus.

Il est entendu que le terme SEAoff signifie le disulfure cyclique non réactif, par opposition au disulfure réactif de structure -N[(CH₂)₂-SH]₂ ci-après appelé SEAon.

Il est entendu que l'acide aminé portant une fonction thiol représenté par Cᵢ₋₁ peut être notamment choisi parmi la cystéine, l'homocystéine, et que ces acides aminés portent un reste disulfure (SR') sur le thiol de l'amino-acide, dans la mise en oeuvre de la réaction avec le peptide-thioester.

Selon l'invention, le procédé conduit à la préparation d'un assemblage peptidique multiple comportant des restes d'acides aminés portant une fonction thiol, c'est à dire un assemblage peptidique de n fragments et de n-1 acides aminés portant une fonction thiol, représenté par la formule :

A₁-C₁-A₂-C₂-A₃- ... -Cᵢ₋₁-Aᵢ-.... -Cₙ₋₁-Aₙ (I)

dans laquelle A₁, A₂, A₃, ... Aᵢ..., Aₙ sont des fragments peptidiques, C₁, C₂, C₃ ... Cᵢ₋₁ ... Cₙ₋₁ sont des restes d'acides aminés portant une fonction thiol,
n est compris entre 3 et 50,
de préférence entre 3 et 20,
ou de manière encore préférée entre 3 et 10, et
i est un nombre entier quelconque compris entre 2 et n.

Il est entendu que, lorsque n = 3, Cₙ₋₁-Aₙ représente C₂-A₃ et Cᵢ₋₁-Aᵢ représente C₁-A₂-Dans ce cas l'assemblage peptidique de formule (I) est de structure : A₁-C₁-A₂-C₂-A₃.

Le procédé selon l'invention consiste dans la préparation d'un peptide-thioester de formule :

A₁-SR (II)

dans laquelle A₁ est un fragment peptidique et SR est le reste d'un thioester alkylé, R pouvant être un radical alkyle éventuellement substitué,
à partir d'un bis(2-sulfanylethyl)amino peptide A₁-SEAoff dans laquelle SEAoff est défini comme précédemment,
par action d'un thiol R-SH, en présence d'un agent réducteur de disulfures cycliques,
suivie de la condensation sur un fragment peptidique de structure :

H-C₁(SR')-A2-SEAoff (III)

dans laquelle C₁, A₂ et SEAoff sont définis comme ci-dessus et (SR') représente un reste disulfure sur le thiol de l'amino-acide C₁, en présence d'un thiol aromatique ArSH,
puis transformation du nouveau fragment peptidique obtenu, de structure :

A₁-C₁-A₂-SEAoff (IV)

dans laquelle A₁, C₁, A₂ et SEAoff sont définis comme précédemment, en un peptide-thioester de formule :

A₁-C₁-A₂-SR (II')

dans laquelle A₁, C₁, A₂ et R sont définis comme précédemment, par action d'un thiol R-SH, en présence d'un agent réducteur de disulfures cycliques,
suivie de la condensation sur un fragment peptidique de structure :

H-C₂(SR')-A₃-SEAoff (III')

dans laquelle SEAoff et R' et C₂ et A₃ sont définis comme ci-dessus, en présence d'un thiol aromatique ArSH, pour donner un fragment peptidique, de structure :

A₁-C₁-A₂-C₂-A₃-SEAoff (IV')

dans laquelle A₁, C₁, A₂, C₂, A₃ et SEAoff sont définis comme précédemment,
puis réitération de ces 2 opérations jusqu'à n-2 fois, pour obtenir un fragment peptidique de structure :

A₁-C₁-A₂-C₂-A₃- ... -Cᵢ₋₁-Aᵢ-... -Cₙ₋₂-Aₙ₋₁-SEAoff (IV^{m})

dans laquelle A₁, A₂, A₃, ... Aᵢ..., Aₙ₋₁, C₁, C₂, C₃ ... Cᵢ₋₁ ... Cₙ₋₂ et SEAoff sont définis comme précédemment, et
mise en oeuvre d'une réaction de ligation native de ce fragment peptidique (IV^{m}) obtenu, avec un peptide de formule :

H-Cₙ₋₁-Aₙ (Vⁿ)

dans laquelle Cₙ₋₁ et Aₙ sont définis comme précédemment, par réduction en présence d'un agent réducteur de disulfures cycliques, pour donner l'assemblage peptidique multiple de formule générale (I).

### DESCRIPTION DE MODES DE REALISATION DE L'INVENTION

L'invention est maintenant décrite plus en détails et de façon non limitative dans la description qui suit.

Par « peptides ou polypeptide » on entend, dans le cadre de la présente demande, une chaîne linéaire de résidus d'acides aminés (en nombre supérieur ou égal à deux) reliés par des liaisons peptidiques. Les « peptides ou polypeptides » au sens de la présente demande peuvent donc par exemple être des oligopeptides, peptides ou protéines selon l'acception conventionnelle de ces termes. Les résidus d'acides aminés présents dans les polypeptides selon l'invention peuvent être choisis parmi les résidus d'acides aminés protéinogéniques ou non. De préférence, ils sont choisis parmi les vingt résidus d'acides aminés protéinogéniques.

La notation des polypeptides s'effectue de l'extrémité N-terminale vers l'extrémité C-terminale. Les résidus d'acides aminés présents le long de la chaîne polypeptidique sont notés selon le code usuel à une lettre ou à trois lettres. Un résidu d'acide aminé est un fragment de polypeptide de formule -NH-(CH-R)-(C=O)-, dans laquelle R représente une chaîne latérale, différente d'un acide aminé à un autre.

Par « fragment peptidique » on entend, dans le cadre de la présente demande, une portion de polypeptide comprenant au moins un résidu d'acide aminé. Un fragment peptidique, au sens de la présente demande, peut donc être par exemple : une séquence de résidus d'acides aminés (telle que -AHG- ou -Ala-His-Gly-) si le fragment peptidique ne comprend ni l'extrémité N-terminale ni l'extrémité C-terminale du polypeptide ; ou bien une séquence de résidus d'acides aminés présentant un groupement à son extrémité N-terminale (telle que H-AHG- ou H-Ala-His-Gly-) si le fragment peptidique comprend l'extrémité N-terminale du polypeptide ; ou bien une séquence de résidus d'acides aminés présentant un groupement à son extrémité C-terminale (telle que -AHG-OH ou -Ala-His-Gly-OH) si le fragment peptidique comprend l'extrémité C-terminale du polypeptide.

Il est entendu que chacun des fragments peptidiques comprend de préférence uniquement des résidus d'acides choisis parmi les 20 résidus d'acides aminés protéinogéniques. Toutefois, selon un mode de réalisation particulier, les fragments peptidiques Aᵢ en position interne de la séquence peuvent aussi comprendre un ou plusieurs résidus d'acides aminés non-protéinogéniques, l'un ou plusieurs des fragments peptidiques A₂ ... Aᵢ ... Aₙ₋₁ pouvant porter un ou plusieurs amino acides modifiés, la modification étant mise en place préalablement à la mise en oeuvre du procédé. A titre d'exemple, non limitatif, la modification des amino acides peut être notamment choisie parmi des radicaux choisis parmi des résidus d'acides carboxyliques (biotine, groupement acétyle, résidu aminooxyacétique, un fluorophore comme la tetraméthylrhodamine, un agent de chélation des métaux comme l'acide 1,4,7,10-tétraazacyclododecane-1,4,7,10-tétraacétique (DOTA), un lipide comme l'acide palmitique, un polymère comme par exemple l'alpha-méthoxy-omega-carboxy poly(éthylène glycol) ou d'autres. Ou encore parmi des modifications post-traductionnelles d'acides aminés protéinogéniques connues de l'homme de l'art (méthylation, phosphorylation, acétylation, glycosylation, sulfatation, hydroxylation, carboxyméthylation, portant les protections appropriées pour l'incorporation en phase solide, etc...), ou éventuellement un médicament (la protéine servant alors de vecteur).

Selon une manière simple, l'introduction d'une modification peut être effectuée en utilisant un acide aminé non-protéinogénique, notamment un dérivé d'acide aminé protéinogénique, pour introduire la modification lors de la synthèse en phase solide du fragment considéré. A titre d'exemple il est possible d'utiliser une Fmoc-L-Lys(Biotine)-OH, c'est à dire une lysine portant sur sa chaîne latérale une biotine, pour effectuer la synthèse d'un fragment. Ce fragment est ensuite engagé dans l'assemblage de manière identique à celle qui emploie les fragments d'acides aminés protéinogéniques.

Selon un mode de réalisation de l'invention, un fragment peptidique Aᵢ comprend entre 2 et 300 résidus d'acides aminés, de préférence entre 5 et 100 résidus d'acides aminés, de manière plus particulièrement préférée entre 8 et 50 résidus d'acides aminés.

Le radical alkyle représenté par R est un radical alkyle de 1 à 12 atomes de carbone, linéaire ou ramifié, éventuellement substitué par un ou plusieurs groupements choisis parmi les atomes d'halogène (F par exemple), ou les radicaux CO₂H, SO₃H, CONH₂, OH, SH, alkyloxy, alkylthio, mercaptoalkylthio, le reste d'un ester ou un reste polyéthylène glycol, ou par un phényle éventuellement substitué, ou d'autres groupements organiques qui n'interfèrent pas avec les réactions mises en oeuvre. Le thiol de formule générale R-SH peut être par exemple HSCH₂CH₂CO2H, HSCH₂CH₂SO3H, HSCH₂CH₂SH, HSCH₂CH₂SCH₂CH₂SH ou HSCH₂Ph.

Le thiol aromatique est avantageusement choisi parmi des composés réducteurs des liaisons disulfure, de préférence choisi parmi le thiophenol et ou les dérivés obtenus par substitution du noyau aromatique, par exemple le 4-carboxymethylthiophénol, l'acide 4-mercaptophénylacétique, le dithiothréitol, le benzylmercaptan et les mélanges de ceux-ci.

Le réducteur de SEAoff peut être choisi parmi les réducteurs de disulfures cycliques. Il peut être notamment choisi parmi les phosphines (par exemple la tris (2-carboxyéthyl)phosphine), ou tout autre réducteur des disulfures cycliques comme les thiols (par exemple le dithiothreitol (DTT)).

Un exemple de peptide A₁-SEAoff peut être représenté par la formule : 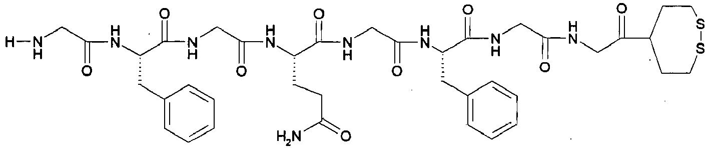 dans cet exemple, le fragment peptidique A₁ est H-GFGQGFGG.

Il est entendu que le fragment peptidique Aₙ peut avoir été préparé préalablement par une suite d'opérations telles que décrites ci-dessus, selon le procédé de l'invention.

Selon l'invention, on peut représenter le procédé d'assemblage de multiples fragments peptidiques selon le schéma 1 suivant :

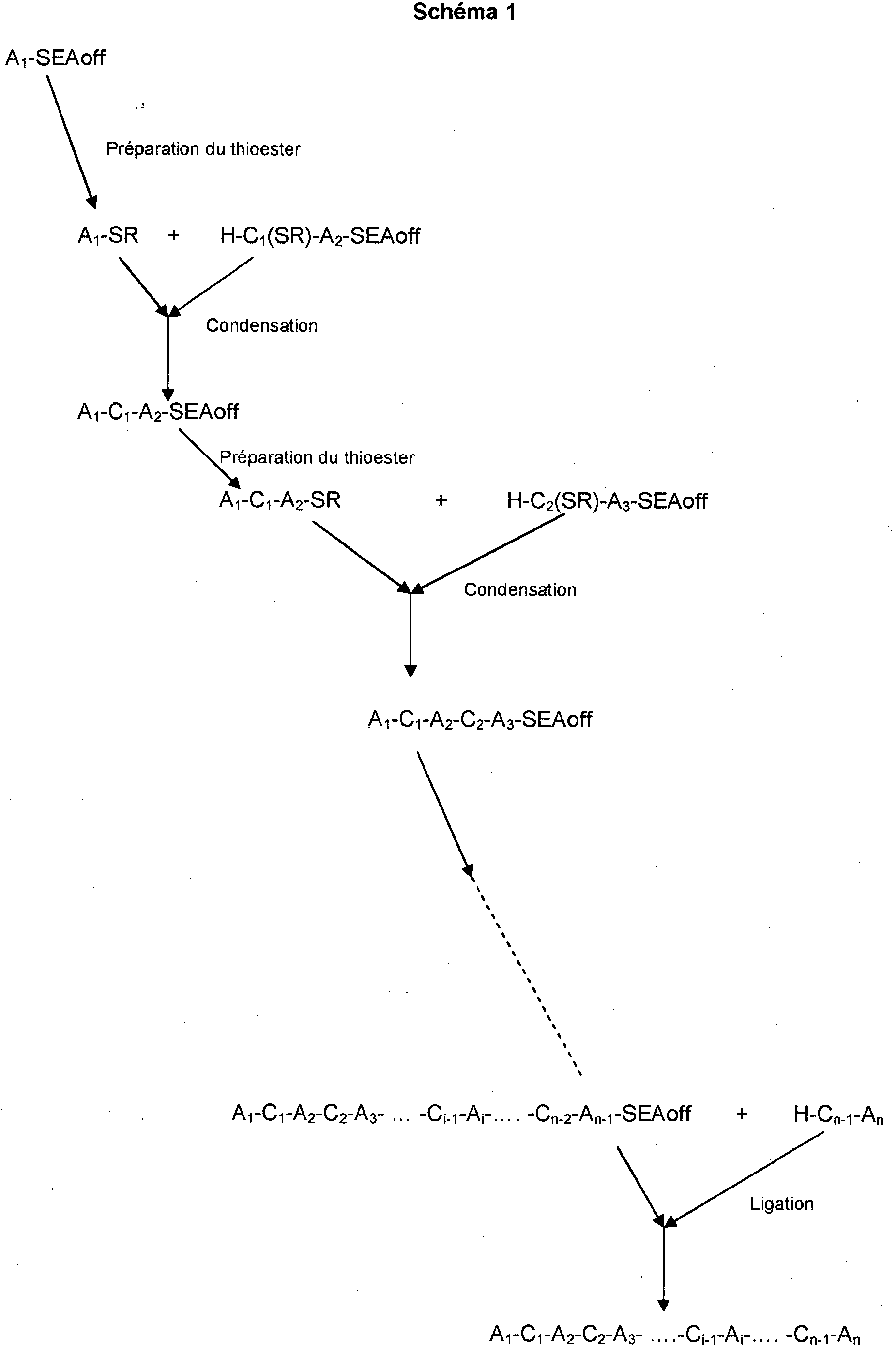

L'ensemble des réactions de préparation de l'assemblage peptidique multiple présente le grand avantage de pouvoir être mis en oeuvre in situ, en une réaction « one pot », particulièrement lorsque le nombre de fragments peptidiques n est égal à 3, sans qu'il soit nécessaire d'isoler les intermédiaires formés.

La réalisation du procédé selon l'invention peut être effectuée comme décrit ci-après, et comme illustré dans les exemples qui suivent.

La préparation des peptides thioesters de formule (II) est avantageusement mise en oeuvre par action d'un alkylthiol dont la partie alkyle est éventuellement substituée sur un bis(2-sulfanyléthyl)amino peptide : A₁-SEAoff, en présence d'un réducteur de disulfures cycliques comme notamment une phosphine, par exemple la tris(2-carboxyethyl)phosphine. La réaction s'effectue avantageusement sous atmosphère inerte (sous argon par exemple), à un pH compris entre 1 et 8 de préférence à pH 4 et à une température comprise entre 20 et 50°C. On utilise avantageusement l'acide 3-mercaptopropionique.

La condensation du thioester de formule (II) sur le fragment peptidique de formule générale (III) : H-C₁(SR')-A₂-SEAoff, s'effectue avantageusement en présence d'un thiol aromatique (notamment l'acide 4-mercaptophénylacétique), en opérant sous atmosphère inerte (sous argon par exemple), à une température comprise entre 20 et 60°C. Il n'est pas nécessaire d'isoler le fragment peptidique obtenu, pour le mettre en oeuvre dans la réaction suivante.

La réaction de ligation native du fragment peptidique (IV^{m}) (A₁-C₁-A₂-C₂-A₃-..-Cᵢ₋₁-Aᵢ-..-Cₙ₋₂-Aₙ₋₁-SEAoff) avec le peptide (Vⁿ) (H-Cₙ₋₁-Aₙ) est mise en oeuvre par action d'un agent réducteur de disulfures cycliques, qui peut être de préférence une phosphine (par exemple la tris(2-carboxyéthyl)phosphine (TCEP)) un composé thiol tel que l'acide 4-mercaptophénylacétique (MPAA), le dithiothréitol (DTT), le thiophénol (et ses dérivés), un alkylthiol (notamment le benzylmercaptan) ou encore des mélanges de plusieurs de ces composés, par exemple MPAA et TCEP, en opérant sous atmosphère inerte (sous argon par exemple), à une température comprise entre 20 et 60°C, de préférence à une température de 37°C. La réaction s'effectue notamment en milieu aqueux, par exemple dans un tampon phosphate., Elle est effectuée, de préférence, à un pH compris entre 5,5 et 8,5, de manière plus particulièrement préférée à un pH compris entre 6 et 8 et idéalement à un pH voisin de 7. La durée de la réaction est fonction des réactifs employés, elle est suivie et ajustée selon les résultats d'une analyse de chromatographie liquide. Il n'est pas nécessaire d'isoler le fragment peptidique obtenu (IV^{m}), pour le mettre en oeuvre dans la réaction de ligation native. Il est entendu que la réaction du réducteur de disulfures cycliques peut être mise en oeuvre sur le fragment peptidique de formule (IV^{m}) avant ou simultanément à la réaction avec le peptide de formule (Vⁿ).

Selon un mode de réalisation particulier, les fragments peptidiques de formule (IV^{m}) et (Vⁿ) comprennent un ou plusieurs résidus d'acides aminés non-protéinogéniques.

Les résidus d'acides aminés des polypeptides de formule (IV^{m}) et (Vⁿ) peuvent éventuellement être protégés par des groupements protecteurs des chaînes latérales. La protection et l'élimination des radicaux protecteurs peut être effectuée selon les méthodes connues qui n'altèrent pas le reste de la molécule. Plus particulièrement selon les méthodes décrites par T. Greene et P. Wuts, Protective groups in organic synthesis, 2ème édition, John Wiley & Sons, Inc.

La préparation du bis(2-sulfanyléthyl)amino peptide : A₁-SEAoff peut être effectuée selon la méthode décrite par Melnyk, O. et coll., Bis(2-sulfanyléthyl)amino native peptide ligation., Org. Lett., 12(22), 5238-41(2010). Selon la réalisation de ce procédé, le couplage d'un acide aminé à un support de résine polymère comprend la mise en présence du support de résine polymère avec un halogénure d'acide aminé ou avec un acide aminé et un agent d'activation, choisi de préférence parmi le PyBOP® (benzotriazol-1-yl-oxytripyrrolidinophosphonium hexafluorophosphate), le BOP, le PyBroP® (bromo-tris-pyrrolidino-phosphonium hexafluorophosphate), ou encore l'HATU (2-(1H-7-azabenzotriazol-1-yl)-1,1,3,3-tetramethyl uronium hexafluorophosphate methanaminium) et de manière plus part iculièrement préférée le PyBroP. Cette réalisation est décrite plus en détails ci-après dans les exemples de préparation des fragments peptidiques de départ.

Notamment, les peptides-N(CH₂CH₂SH)₂ (peptides SEAon) correspondants sont oxydés par l'air, sous agitation, dans une solution de bicarbonate d'ammonium 0,1M, à température ambiante. Avantageusement, ils peuvent être aussi obtenus par oxydation en présence d'iode ou de diamide (CH₃)₂NCON=NCON(CH₃)₂.

Le polypeptide de formule (Vⁿ) peut être par exemple obtenu par une méthode de synthèse peptidique usuelle, notamment une méthode de synthèse en phase solide. Il peut également être obtenu au moyen d'une ligation native telle que décrite dans Org. Lett., 12(22), 5238-41 (2010).

Selon des modes préférés de la présente invention, on prépare un assemblage peptidique de 3 ou 4 fragments, de structures A₁-C₁-A₂-C₂-A₃ ou A₁-C₁-A₂-C₂-A₃-C₃-A₄.

Selon un autre mode préféré de l'invention, les amino acides Cᵢ portant une fonction thiol sont des restes cystéine Cys.

Dans ce cas, selon un mode préféré de réalisation de l'invention, on prépare un assemblage peptidique de structure A₁-Cys-A₂-Cys-A₃ ou A₁-Cys-A₂-Cys-A₃-Cys-A₄.

Selon ces modes préférés, le schéma de préparation de l'assemblage peptidique peut être représenté selon le schéma 2 suivant :

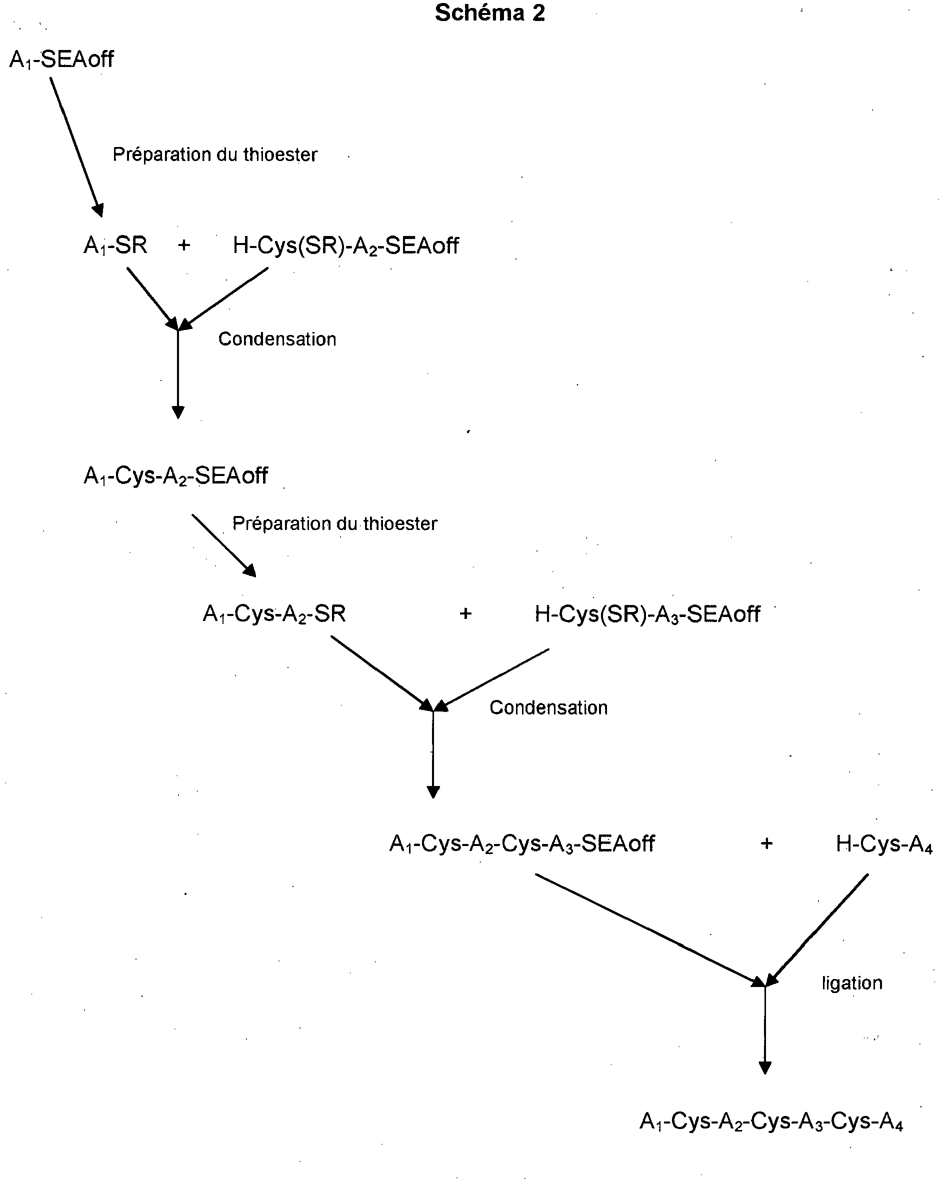

La présente invention concerne également la préparation des peptide-thioesters de formule (II) à partir de bis(2-sulfanylethyl)amino peptide : A₁-SEAoff selon le procédé décrit ci-dessus, par action d'un thiol R-SH, en présence d'un agent réducteur de disulfures cycliques. L'invention concerne également les peptides thioesters ainsi obtenus.

Les polypeptides de formule (I) obtenus selon l'invention peuvent être utilisés pour produire une banque de polypeptides, par exemple à des fins de criblage.

Ils peuvent également être utilisés pour la fabrication de compositions pharmaceutiques, en combinaison avec un ou plusieurs adjuvants ou véhicules compatibles et pharmaceutiquement acceptables. A titre d'exemple de compositions pharmaceutiques pouvant être obtenues selon l'invention, on peut citer les médicaments utilisables chez l'homme ou chez l'animal et les préparations vaccinales.

Ils peuvent également être utilisés pour la réalisation de kits de diagnostic.

Selon un autre objet de la présente invention, l'invention concerne également un procédé de fabrication d'une composition pharmaceutique comprenant :
- la fabrication d'un polypeptide ou peptide selon le procédé de fabrication d'un polypeptide de formule (I) décrit précédemment, et
- les compositions pharmaceutiques comprenant un polypeptide ou peptide ainsi préparé, à l'état pur ou en association avec un ou plusieurs adjuvants compatibles et pharmaceutiquement acceptables.

L'invention concerne également un procédé de fabrication d'un dispositif de diagnostic comprenant :
- la fabrication d'un polypeptide ou peptide selon le procédé de fabrication d'un polypeptide de formule (I) décrit précédemment, et
- la formulation ou la mise en forme de ce polypeptide sous une forme adaptée à une utilisation pour un dispositif de diagnostic.

Le procédé selon la présente invention est particulièrement intéressant du fait qu'il permet de synthétiser, en synthèse totale, un enchaînement peptidique de la longueur et de la nature souhaitée, et comportant des motifs amino acides à substitution thiol du type cystéine (Cys) ou homocystéine, répartis dans la chaîne.

Il présente l'avantage d'être industrialisable, du fait de la synthèse convergente dont il s'agit, et ce type de synthèse est particulièrement recherché en matière d'industrialisation de procédés puisqu'il procure des rendements avantageux, et notamment peu de perte de rendement en produits déjà élaborés dont le coût de synthèse est déjà élevé.

Selon la présente invention les fragments SEA permettent la synthèse de protéines en partant du N-terminal vers le C-terminal par ligation successive de fragments peptidiques.

L'assemblage du N-terminal vers le C-terminal offre un avantage considérable, comparé à la stratégie inverse d'assemblage du C-terminal vers le N-terminal, en effet, dans ce cas le procédé induit une auto-purification des fragments peptidiques ce qui est considérablement important en synthèse industrielle.

Selon un autre avantage le procédé de l'invention permet d'utiliser des acides aminés protéinogéniques pour la synthèse des fragments peptidiques. De ce fait, il est inutile de recourir à la fabrication de dérivés d'acides aminés (tels que des cétoacides par exemple), ce qui alourdirait considérablement la synthèse.

Les exemples qui suivent illustrent la présente invention.

### Exemple 1

### Assemblage one-pot de 3 fragments

### 1- Synthèse du peptide 1 (H-ILKEPVHGA-S(CH₂)₂COOH), équivalent de A₁-SR avec R = alkyle, par transformation de A₁-SEAoff en présence d'un alkylthiol R-SH, R = -(CH₂)₂COOH et d'un réducteur de disulfures cycliques.

Le peptide précurseur H-ILKEPVHGA-SEAoff a été préparé comme décrit par Ollivier N., Dheur J., Mhidia R., Blanpain A., and Melnyk O., Bis(2-sulfanylethyl)amino native peptide ligation., Org. Lett. 12(22), 5238-41 (2010).

Le chlorhydrate de tris(2-carboxyethyl)phosphine TCEP,HCl (921,2 mg) est dissous dans 0,2 M tampon phosphate pH=7,3 (40 mL). L'acide 3-mercaptopropionique (MPA) (2 mL) est ajouté à cette solution. Le pH de la solution est réajusté à 4 avec de la soude 5 N (environ 3 mL).

Le peptide H-ILKEPVHGA-SEAoff (40,31 mg) est dissous dans la solution précédente (40 mL). Le milieu réactionnel est placé sous atmosphère inerte par 3 cycles vide/argon, et placé dans un bain à 37°C.

Après 22 heures, le milieu réactionnel est transféré dans une ampoule a décanter. Une solution aqueuse d'acide trifluoroacétique (TFA) à 7% (20 gouttes) est additionnée. 4 extractions à l'éther diéthylique sont réalisées. Une solution aqueuse de TFA à 7% (10 mL) est additionnée et 3 nouvelles extractions à l'éther diéthylique sont réalisées.

La phase aqueuse est dégazée pendant 13 minutes en faisant buller de l'argon, avant d'être purifiée en RP-HPLC (colonne C18 nucléosil 120Å, 5µm, tampon A 100% eau contenant 0,05% TFA, tampon B CH3CN/eau 4/1 contenant 0,05% TFA, gradient : 0 à 10% de tampon B en 5 mn puis 10 à 100% de tampon B en 150 mn, débit 6 mL/mn, 215 nm). On obtient 18,7 mg de peptide 1 (H-ILKEPVHGA-S(CH2)2COOH) pur (R=47,4%) C₄₇H₇₈N₁₂O₁₃S₁ MALDI-TOF [M+H]⁺ calculé (masse mono isotopique) 1051,56; observé 1051,70.

### 2- Synthèse peptide 2 H-C(StBu)HHLEPGG-SEAoff, équivalent de H-C₁(SR)-A₂-SEAoff, avec R = tBu

Synthèse classique d'un peptide-SEAoff à une échelle de 0,25 mmole, selon :
Ollivier N., Dheur J., Mhidia R., Blanpain A., and Melnyk O., Bis(2-sulfanylethyl)amino native peptide ligation., Org. Lett. 12(22), 5238-41 (2010).

Le reste disulfure -S-t.Bu est couplé au moyen de Fmoc-Cys(St.Bu)-OH, de manière analogue à la méthode décrite dans l'exemple 2 ci-après.

La déprotection finale et la coupure du peptide de la résine sont réalisés avec TFA/TIS/DMS/H20 (90/2,5/2,5/5/2,5 en volume, 25 mL) pendant 1h30. Le peptide est précipité dans un mélange froid éther diéthylique/heptane (1/1 en volume), dissous dans un minimum d'eau et lyophilisé. 132,6 mg de peptide brut sont obtenus (R=38%). C₄₃H₆₉N₁₃O₁₀S₄ MALDI-TOF [M+H]+ calculé (masse monoisotopique) 1056,42; observé 1056,10.

Une partie du peptide 2 est ensuite oxydée avant purification. Pour cela, le fragment 2 (122,6 mg) et I₂ (222,52 mg, 10 éq.) sont dissous dans AcOH/eau 4/1 (12,5 ml). Après 10 minutes d'agitation, le milieu réactionnel dilué (Volume total =37 mL) est transféré dans une ampoule à décanter contenant de l'éther diéthylique (75 mL). 3 extractions à l'éther diéthylique sont réalisées (3 x 75 mL). La phase aqueuse est congelée et lyophilisée pour donner 94,3 mg de peptide brut.

Après purification RP-HPLC (colonne C18 nucléosil 120Å, 5µm, 215 nm, tampon A 100% eau contenant 0,05% TFA, tampon B CH3CN/eau 4/1 contenant 0,05% TFA, gradient: 0 à 10% de tampon B en 5 mn, puis 10 à 100% de tampon B en 150 mn, débit 6 mL/min) on obtient 40,52 mg de peptide pur (R=33%) C₄₃H₆₇N₁₃0₁₀S₄ MALDI-TOF [M+H]+ calculé 1054,4 (masse monoisotopique); observé 1054,4.

### 3) A₁-SR (peptide 1) (R = CH₂CH₂CO₂H) + H-C₁(StBu)-A₂-SEAoff (peptide 2) → A₁-C₁-A₂-SEAoff

L'acide 4-mercaptophénylacétique (MPAA, 33,64 mg, 0,2 mmol, thiol aromatique) est dissous dans du tampon phosphate pH=7,3 0,1 M (1 mL). Une solution de soude 5 N (80 µL) est ajoutée pour ajuster le pH de la solution à 7,64. Le peptide 1 (10,40 mg, 7,5 µmol) et le peptide 2 (10,42 mg, 7,5 µmol) sont dissous ensemble dans la solution précédente (533µL). Le milieu réactionnel est placé dans un bain thermostaté à 37°C sous atmosphère inerte.

La ligation est suivie par RP-HPLC sur une colonne C18 Xbridge BEH (4,6 x 250 mm, 300 Å, 5µm) (215 nm, 1 mUmn, 30°C, tampon A eau contenant 0,05% TFA, tampon B CH3CN/eau 4/1 contenant 0,05% TFA, 0-100% B en 30 mn). Pour cela, un aliquot (5 µL) de milieu réactionnel est acidifié avec une solution de TFA à 10% dans l'eau, extrait avec de l'éther diéthylique pour éliminer le MPAA et le MPA avant l'analyse.

### 4- Synthèse peptide 3 H-CILKEPVHGV-NH2, équivalent de H-C₂-A₃

La préparation du peptide H-C₂-A₃ est décrite dans la publication de : Ollivier N., Dheur J., Mhidia R., Blanpain A., and Melnyk O., Bis(2-sulfanylethyl)amino native peptide ligation., Org. Lett. 12(22), 5238-41 (2010).

### 5- Protocole de ligation one-pot de trois fragments.

### Synthèse peptide 4 H-ILKEPVHGACHHLEPGGCILKEPVHGV-NH2, équivalent de A₁-C₁-A₂-C₂-A₃

### Préparation du peptide 4 à partir de A₁-C₁-A₂-SEAoff et de peptide 3 (H-C₂-A₃)

### A₁-C₁-A₂-SEAoff transformé en A₁-C₁-A₂-SEAon par réduction en présence de TCEP (tris(2-carboxyethyl)phosphine)

Après 5h30 de réaction, la TCEP,HCl (chlorhydrate de tris(2-carboxyethyl)phosphine) (57,34 mg, 0,2 mmol) est dissoute dans un tampon phosphate pH=7,3 0,1 M (1 mL). une solution de soude 5 N (140 µL) est ajoutée pour ajuster le pH de la solution à 7,33.

A₁-C₁-A₂-SEAon + **(peptide 3)** H-C₂-**A₃ → A₁-C₁-A₂-C₂-A₃ (peptide 4)**

Le **peptide 3** (16,17 mg, 11,2 µmol) est dissous dans la solution précédente (533 µL) puis ajouté au milieu réactionnel précédent qui est placé à nouveau à 37°C sous atmosphère inerte.

La ligation est suivie par RP-HPLC sur une colonne CI8 Xbridge BEH (4,6 x 250 mm, 300 A, 5 µm) (215 nm, 1 mL/mn, 30°C, tampon A eau contenant 0,05% TFA, tampon B CH3CN/eau 4/1 contenant 0,05% TFA, 0-100% B en 30 mn). Pour cela, un aliquot (5 µL) de milieu réactionnel est acidifié avec une solution de TFA à 10% dans l'eau, extrait avec l'éther diéthylique pour éliminer le MPAA (acide 4-mercaptophénylacétique) avant l'analyse.

Lorsque la réaction est complète, le milieu réactionnel est dilué avec de l'eau contenant 0,05% de TFA (3 mL) et une solution de TFA à 10% dans l'eau (15 gouttes) est additionnée. Après 5 extractions avec l'éther diéthylique (5 x 8 mL), la phase aqueuse est dégazée pendant 10 minutes par bullage d'argon. Après purification par RP-HPLC sur une colonne C18 Nucléosil 120Å, 5 µm (215 nm, débit 6 mL/mn, tampon A : eau contenant 0,05% TFA, tampon B : CH3CN/eau 4/1 contenant 0,05% TFA, gradient : 0-10% de tampon B en 5 mn, puis 10-100% de tampon B en 150 mn) on obtient 16,2 mg de peptide 4 pur (59,2%).

### Exemple 2

### 1-Synthèse de K1(fragment 1)-MPA

### a-Synthèse de K1(fragment 1)-SEAoff

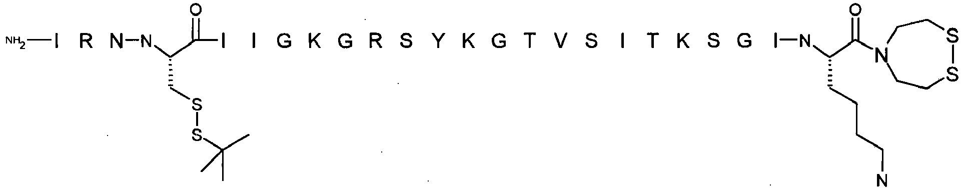

La résine SEA (0,5 mmol, 0,175 mmol/g) est conditionnée dans le dichlorométhane (DCM). Fmoc-Lys(Boc)-OH (2,342 g, 5 mmol) est dissous dans du DCM et quelques gouttes de diméthylformamide (DMF) pour aider à la solubilisation puis la solution est ajoutée sur la résine. PyBrop (2,331 g, 5 mmol) est dissous dans le minimum de DCM puis ajouté sur la résine. La diisopropyléthylamine (DIEA) (2,613 mL, 15 mmol) est ensuite additionnée sur la résine et le couplage dure 2 heures (volume total <5 mL). La résine est ensuite lavée 3 x 2 minutes avec du DCM. La résine est ensuite traitée par 10% Ac₂O/5% DIEA/DCM (10 mL, 2 min) puis (10 mL, 20 min). La résine est ensuite lavée 5 x 2 minutes au DCM.

Le fragment 1 est assemblé sur une partie de la résine précédente (0,25 mmol, 0,175 mmol/g) avec un synthétiseur de peptides (CEM µWaves, Saclay, France) sans utiliser les micro-ondes, en utilisant la stratégie Fmoc/*tert*-butyle. Le couplage est effectué avec les acides aminés (0,2 M, 4 éq), l'activateur HBTU (0,5 M, 3,6 éq.) et la base DIEA (2 M, 8 éq). La déprotection finale et le clivage du peptide de la résine sont réalisés avec TFA/TIS/DMS/thioanisole/H₂O (90/2,5/2,5/2,5/2,5 en volume, 25 mL) pendant 2 heures 30. Le peptide est précipité dans un mélange froid éther diéthylique/heptane (1/1 en volume), dissout dans un minimum d'eau et lyophilisé. 288 mg de peptide brut sont obtenus (R=32%). C₁₂₀H₂₁₆N₃₆O₃₁S₄ LC-MS [M+H]⁺ calculé (masse moyenne) 2788,5; observé 2788,1.

Une partie du fragment 1 est ensuite oxydée avant purification. Pour cela, le fragment 1 (95,7 mg) est dissous dans AcOH/eau 1/4 (47,9 mL). 197 mM l₂ dans DMSO (270 µL, 50 µmol, 2éq.) sont additionnées à la solution précédente. Après 30 secondes d'agitation, 64,8 mM dithiothréitol (DTT, 823 µL, 50 µmol, 2 éq) est additionné pour consommer l₂ restant et injecté directement en RP-HPLC.

Après purification RP-HPLC (colonne C18 nucléosil 120Å, 5µm, tampon A 100% eau contenant 0,05% TFA, tampon B CH₃CN/eau 4/1 contenant 0,05% TFA, gradient : 0 à 20% de tampon B en 5 min, puis 20 à 40% en 40 min, débit 6 mL/min, 215 nm) on obtient 25,8 mg de peptide pur (R=27%) C₁₂₀H₂₁₄N₃₆O₃₁S₄ MALDI-TOF [M+H]⁺ calculé (masse monoisotopique) 2784,52 ; observé 2784,6.

### b-Synthèse de K1 (fragment 1)-MPA

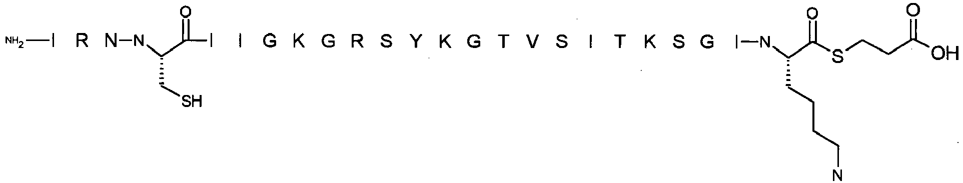

Le *Tris*(2-carboxyethyl)phosphine hydrochlorure (TCEP,HCl, 229,63 mg, 0,8 mmol) est solubilisé dans le tampon 0,2 M phosphate de sodium pH=7,3 (10 mL). L'acide mercaptopropionique (MPA, 0,5 mL) est additionné. NaOH 5M (340 µL) est ajouté pour ajuster le pH à 3,98.

Le fragment 1 K1-SEAoff (24,67 mg, 6,9 µmol) est dissous dans la solution précédente (10,3 mL). Le milieu réactionnel est chauffé à 37°C sous atmosphère d'argon. L'avancement de la réaction est suivi par RP-HPLC sur une colonne C18 Xbridge BEH (215 nm, 1 mL/min, 30°C, éluant A eau contenant 0,05% d'acide trifluoracétique (TFA), éluant B CH₃CN/eau 4/1 contenant 0,05% TFA, 0-100% B en 30 min). Pour cela, des aliquots sont acidifiés avec 10 % de TFA aqueux, extraits avec Et₂O afin d'éliminer le MPA en excès avant analyse.

Après la fin de la réaction, le milieu réactionnel est dilué par de l'eau (10 mL) et 10 % de TFA aqueux (5 mL) sont additionnés. Après 3 extractions avec Et₂O (3 × 15 mL) et bullage d'argon pendant 15 min, la purification par RP-HPLC sur une colonne C18 Nucleosil 120 Å, 5 µm (215 nm, 6 mL/min, température ambiante, éluant A eau contenant 0,05% TFA, éluant B CH3CN/eau 4/1 contenant 0,05% TFA, 0-10% B en 5 min, puis 10-100% B en 150 min) fournit 9,7 mg de K1(fragment 1)-MPA pur (41%).

### 2-Synthèse de StBu-K1(fragment 2)-SEAoff

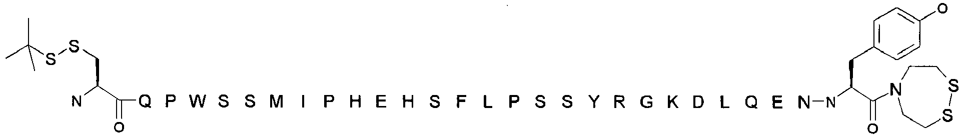

La résine SEA (0,5 mmol, 0,175 mmol/g) est conditionnée dans du DCM. Fmoc-Tyr-OH (2,298 g, 5 mmol) est dissous dans du DCM (<5 mL) et quelques gouttes de DMF pour aider à la solubilisation puis ajouté sur la résine. PyBrop (2,331 g, 5 mmol) est dissous dans le minimum de DCM puis ajouté sur la résine. DIEA (2,614 mL, 15 mmol) est ensuite additionné sur la résine et le couplage dure 2 heures. La résine est ensuite lavée 4 x 2 minutes au DCM. La résine est ensuite traitée par 10% Ac₂O/5% DiEA/DCM (10 mL, 2 min) puis (10 mL, 20 min). La résine est ensuite lavée 5 x 2 minutes au DCM.

Le fragment 2 est assemblé sur la résine précédente (0,5 mmol, 0,175 mmol/g) avec un synthétiseur de peptides (CEM µWaves, Saclay, France) sans utiliser les micro-ondes, en utilisant la stratégie Fmoc/*tert*-butyle. Le couplage est effectué avec les acides aminés (0,2 M, 4 éq.), l'activateur HBTU (0,5 M, 3,6 éq.) et la base DIEA (2 M, 8 éq.). Le solvant de lavage (DMF) ainsi que le solvant de Fmoc-Met-OH contiennent 1% de thioanisole afin d'éviter au maximum l'oxydation de la méthionine de la séquence.

La résine est séparée en 2 après la glutamine en position 2 (0,25 mmol) afin de coupler la Fmoc-Cys(StBu)-OH manuellement. Pour ce faire, la résine est lavée 4 x 2 minutes au DMF, pesée en DMF et divisée en 2. HBTU (379,3 mg, 1 mmol) est dissous dans DMF (1100 µL). HOBt (135 mg, 1 mmol) est dissous dans DMF (500 µL) et additionné sur HBTU. Fmoc-Cys(StBu)-OH (431,6 mg, 1 mmol) est dissous dans DMF (500 µL) et additionné au mélange HBTU/HOBt. DIEA (522,7 µL, 3 mmol) est ensuite ajouté sur le mélange. On agite pendant 1 minute puis le mélange est additionné sur la résine et le couplage dure 45 minutes. La résine est ensuite lavée 4 x 2 minutes au DMF. On réalise la déprotection du Fmoc N-terminal par traitement à la pipéridine 20% dans le DMF (15 mn puis 5 mn). La résine est ensuite lavée 4 x 2 minutes au DCM puis 3 x 2 minutes à Et₂O et séchée.

La déprotection finale et le clivage du peptide de la résine sont réalisés avec TFA/TIS/DMS/Thioanisole/H₂O (90/2,5/2,5/2,5/2,5 en volume, 25 mL) pendant 2 heures 30. Le peptide est précipité dans un mélange froid éther diéthylique/heptane (1/1 en volume), dissous dans un minimum d'eau et lyophilisé. 366,14 mg de peptide brut sont obtenus (R=35,6%). C₁₅₆H₂₃₁N₄₁O₄₄S₅ MALDI-TOF [M+H]⁺ calculé (résolution monoisotopique) 3543,6; observé 3542,2.

Une partie du fragment 2 est ensuite oxydée avant purification. Pour cela, le fragment 2 (67 mg) est dissous dans AcOH/eau 1/4 (35 mL) puis 144 mM I₂ dans DMSO (223 µL) sont ajoutés. Après 30 secondes d'agitation, 64,8 mM DTT dans AcOH/eau 1/4 (503 µL) sont additionnés pour consommer l'I₂ en excès. Après décoloration de la solution, le milieu est injecté en RP-HPLC (colonne C18 nucléosil, 120 A, 5 µm, 2 x 28 cm, 215 nm, tampon A 100% eau contenant 0,05% TFA, tampon B CH₃CN/eau 4/1 contenant 0,05% TFA, gradient : 0 à 10% de tampon B en 5 min, puis 10 à 100% B en 150 min, débit 6 mL/min) on obtient 11,5 mg de peptide pur (R=17,2 %) C₁₅₆H₂₂₉N₄₁O₄₄S₅ LC-MS [M+H]⁺ calculé (résolution monoisotopique) 3541,6 ; observé 3541,9.

### 3-Synthèse de K1(fragment 3)

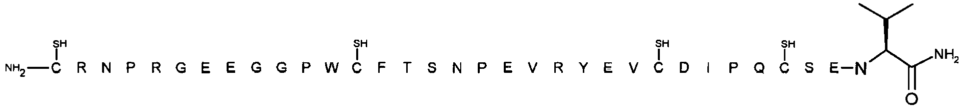

Le fragment 3 est assemblé sur la résine Novasyn TGR (0,5 mmol, 0,25 mmol/g) avec un synthétiseur de peptides (CEM µWaves, Saclay, France) sans utiliser les micro-ondes, en utilisant la stratégie Fmoc/*tert*-butyle. Le couplage est effectué avec les acides aminés (0,2 M, 4 éq.), l'activateur HBTU (0.5 M, 3.6 éq.) et la base DIEA (2 M, 8 éq.).

La déprotection finale et le clivage du peptide de la résine sont réalisés avec TFA/EDT/H₂O/TIS (94/2,5/2,5/1 en volume, 30mL) pendant 2 heures 30. Le peptide est précipité dans un mélange froid éther diéthylique/heptane (1/1 en volume), dissous dans un minimum d'eau et lyophilisé. Après purification RP-HPLC (colonne C18 vydac 50 cm x 2 cm, 280 nm, tampon A 100% eau contenant 0,05% TFA, tampon B CH₃CN/eau 4/1 contenant 0.05% TFA, gradient : 0 à 20% de tampon B en 10 min puis 20 à 50% de tampon B en 60 mn, débit 30 mL/mn) on obtient 272 mg de peptide pur (R=13%) C₁₅₈H₂₃₉N₄₇O₅₂S₄ MALDI-TOF [M+H]⁺ calculé (résolution monoisotopique) 3755,6; observé 3755,7.

### 4-Protocole de ligation one-pot :

### Synthèse d'un peptide 4 K1 (125-209)

La guanidine HCl (573,24 mg, 6 mmol) est dissoute dans 0,1 M tampon phosphate pH=7,3 (1 mL). L'acide 4-mercaptophenylacétique (MPAA, 33,68 mg, 0,2 mmol) est dissous dans cette dernière solution (1 mL). NaOH 5M (150 µL) est ajouté pour ajuster le pH de la solution à 7,53.

Le K1 (fragment 1)-MPA (9,71 mg, 2,8 µmol) et le StBu-K1(fragment 2)-SEA(10,65 mg, 2,6 µmol) sont dissous ensemble dans la solution précédente (185 µL). Le milieu réactionnel est placé dans un bain thermostaté à 37°C dans la boîte à gants.

La ligation est suivie par RP-HPLC sur une colonne C18 Xbridge BEH (4,6 x 250 mm, 300 A, 5 µm) (215 nm, 1 mL/min, 30°C, tampon A eau contenant 0,05% TFA, tampon B CH₃CN/eau 4/1 contenant 0,05% TFA, 0-100% B en 30 min). Pour cela, un aliquot (1 µL) de milieu réactionnel est acidifié avec 10% TFA aqueux, extrait avec l'Et₂O pour éliminer le MPAA et MPA avant l'analyse.

Après 20 heures de réaction, de la guanidine HCl (573,15 mg, 6 mmol) est dissoute dans du tampon phosphate 0,1 M, pH=7,3 (1 mL). Le *Tris*(2-carboxyéthyl) phosphine hydrochlorure (TCEP,HCl, 57,59 mg, 0,2 mmol) est dissous dans cette dernière solution (1 mL). 5M NaOH est ajouté pour ajuster le pH de la solution à 7,27.

Le K1(fragment 3) (16,46 mg, 3,9 µmol) est dissous dans la solution précédente (185 µL) puis ajouté au milieu réactionnel précédent qui est placé à nouveau à 37°C sous atmosphère inerte.

La ligation est suivie par RP-HPLC sur une colonne C18 Xbridge BEH (4,6 x 250 mm, 300 A, 5 µm) (215 nm, 1 mL/min, 30°C, tampon A eau contenant 0,05% TFA, tampon B CH₃CN/eau 4/1 contenant 0,05% TFA, 0-100% B en 60 min). Pour cela, un aliquot (1 µL) de milieu réactionnel est acidifié avec 10% TFA aqueux, extrait avec Et₂O pour éliminer le MPAA et MPA avant l'analyse.
C₄₁₈H₆₄₈N₁₂₂O₁₂₇S₇ MALDI-TOF [M+H]⁺ (masse moyenne) calculé 9640.01, trouvé 9639.

Lorsque la réaction est complète, le milieu réactionnel est dilué avec de l'eau (4 mL) et TFA 10% aqueux (1 mL) est additionné. Après 4 extractions avec Et₂O (4 x 8 mL), la phase aqueuse est dégazée pendant 10 minutes par bullage d'argon, diluée avec de l'eau (36 mL), filtrée. Le fritté est rincé par AcOH (1 mL) et rincé par de l'eau (20 mL). L'ensemble est purifié par RP-HPLC sur une colonne C18 vydac 300 Å, 5 µm (215 nm, 20 mL/min, tampon A eau contenant 0,05% TFA, tampon B CH3CN/eau 4/1 contenant 0,05% TFA, 0-20% B en 5 min, puis 20-60% B in 80 min) on obtient 11,46 mg de K1 (125-209) pur (R = 39,4%).

### 5-Folding de K1(125-209)

K1(125-209) (3,99 mg, 0,355 µmol) est dissous dans le tampon 10 mM PBS, 138 mM NaCl, 2,7 mM KCI pH=7,4 contenant 10% en volume de glycérol, 1 mM glutathion réduit, 0,2 mM glutathion oxydé (10 mL).

Le milieu réactionnel est placé à 4°C.

Le folding est suivi par LC-MS sur une colonne C18 Xbridge BEH (4,6 x 250 mm, 300 A, 5 µm) (215 nm, 1 mL/min, 30°C, tampon A eau contenant 0,1 % TFA, tampon B CH₃CN/eau 4/1 contenant 0,1 % TFA, 0-100% B en 60 min).

Après 65 heures, le milieu réactionnel est ultracentrifugé à 8000 rcf, à 4°C pendant 15 minutes puis à 9000 rcf, à 4°C pendant 15 minutes afin d'éliminer quelques agrégats.

Le milieu réactionnel (7,6 mL) est ensuite transféré en plusieurs fois dans un système d'ultrafiltration vivaspin 2mL avec un cut-off de 3000 MWCO et ultrafiltré à 12000 rcf, à 12°C pendant 3 heures 20. Une fois concentré, le milieu réactionnel est de 800 µL environ.

Il est ensuite dialysé à 4°C dans une cassette de dialyse de cut-off 3500 MWCO avec un tampon 10 mM PBS, 138 mM NaCl, 2,7 mM KCI pH=7,4 contenant 10% en volume de glycérol (1 L) pendant 1 nuit. Le milieu réactionnel est de 1 mL environ après dialyse.

K1 (125-209) foldé est dosé à 562 nm par le kit BCA Protein Assay (Pierce) en réalisant une gamme étalon de BSA. La concentration estimée de K1 (125-209) est de 137 µM.

### Exemple 3

### 1-Synthèse de K1 (fragment 3)-biotine

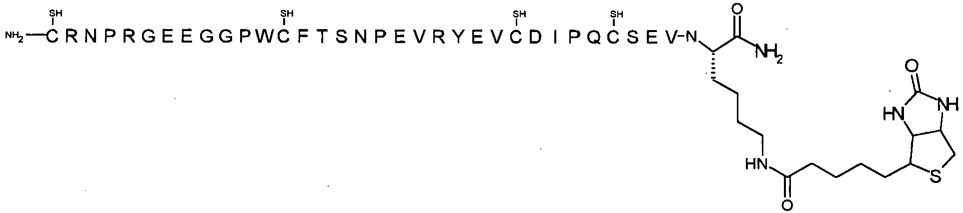

Fmoc-Lys(Biotine)-OH (2 × 89 mg, 2 × 0,15 mmol) est couplé 2 fois sur la résine PAL chemmatrix (0,1 mmol, 0,43 mmol/g) en utilisant TBTU (2 × 30,5 mg, 2 × 0,15 mmol) et DIEA (2 × 50 µL, 2 × 0,45 mmol) pendant 2 heures dans le NMP. Après des lavages au NMP (3 x 2 min) et DM F (3 x 2 mn), le fragment 3-biotine est assemblé sur une 2 colonnes de 50 µmol avec un synthétiseur de peptides (INTAVIS) en utilisant la stratégie Fmoc/*tert-*butyle.

Les couplages sont effectués en utilisant 4 éq. de chaque acide aminé, 3,6 éq. HBTU, et 8 éq. DIEA. Une étape de capping est réalisée après chaque couplage avec Ac₂O/DIEA. A la fin de la synthèse, la résine est lavée avec CH₂Cl₂, diéthyl éther (2 × 2 min) et séchée sous vide.

La déprotection finale et le clivage du peptide de la résine sont réalisés avec TFA/EDT/H₂O/TIS (94/2,5/2,5/1 en volume, 2 × 5mL) pendant 2h. Le peptide est précipité dans un mélange froid éther diéthylique/heptane (1/1 en volume), dissout dans un minimum d'eau et lyophilisé. Après purification RP-HPLC (colonne C18 nucléosil 50 cm x 2 cm, 215 nm, tampon A 100% eau contenant 0,05% TFA, tampon B CH₃CN/eau 4/1 contenant 0,05% TFA, gradient : 0 à 15% de tampon B en 5 min puis 15 à 35% de tampon B en 60 min, débit 6 mL/min) on obtient 36,75 mg de peptide pur (Rdt=8 %)
C₁₇₄H₂₆₅N₅₁O₅₅S₅ MALDI-TOF [M+H]⁺ calculé (masse moyenne) 4112,68; observé 4114,3.

### 2-Protocole de ligation one-pot :

### synthèse d'un peptide 4 K1(125-209) K-biotine

La guanidine HCl (573,28 mg, 6 mmol) est dissoute dans 0,1 M tampon phosphate pH=7,3 (1 mL). L'acide 4-mercaptophenylacétique (MPAA, 33,59 mg, 0,2 mmol) est dissous dans cette dernière solution (1 mL). NaOH 5M (80 µL) est ajouté pour ajuster le pH de la solution à 7,40.

Le K1(fragment 1)-MPA (2,76 mg, 0,8 µmol) et le StBu-K1(fragment 2)-SEA (3,30 mg, 0,8 µmol) sont dissous ensemble dans la solution précédente (57 µL). Le milieu réactionnel est placé dans un bain thermostaté à 37°C dans la boîte à gants.

La ligation est suivie par RP-HPLC sur une colonne C18 Xbridge BEH (4,6 x 250 mm, 300 Å, 5µm) (215 nm, 1 mL/min, 30°C, tampon A eau contenant 0,05% TFA, tampon B CH₃CN/eau 4/1 contenant 0,05% TFA, 0-100% B en 30 min). Pour cela, un aliquot (1 µL) de milieu réactionnel est acidifié avec 10% TFA aqueux, extrait avec Et₂O pour éliminer MPAA et MPA avant l'analyse.

Après 24h40 de réaction, de la guanidine,HCl (573,18 mg, 6 mmol) est dissoute dans du tampon phosphate 0,1 M, pH=7,3 (1 mL). Le *Tris*(2-carboxyéthyl)phosphine hydrochlorure (TCEP,HCl, 57,80 mg, 0,2 mmol) est dissous dans cette dernière solution (1 mL). NaOH 5M est ajouté pour ajuster le pH de la solution à 7,40.

Le K1(fragment 3) (5,50 mg, 1,2 µmol) est dissous dans la solution précédente (57 µL) puis ajouté au milieu réactionnel précédent qui est placé à nouveau à 37°C sous atmosphère inerte.

La ligation est suivie par RP-HPLC sur une colonne C18 Xbridge BEH (4,6 x 250 mm, 300 Å, 5 µm) (215 nm, 1 mL/min, 30°C, tampon A eau contenant 0,05% TFA, tampon B CH₃CN/eau 4/1 contenant 0,05% TFA, 0-100% B en 60 min). Pour cela, un aliquot (1 µL) de milieu réactionnel est acidifié avec 10% TFA aqueux, extrait avec Et₂O pour éliminer MPAA et MPA avant l'analyse.
C₄₃₄H₆₇₄N₁₂₆O₁₃₀S₈ [M+H]⁺ attendu (masse moyenne) 9994,49, observé 9994,41.

A la fin de la réaction, le milieu réactionnel a été dilué avec de l'eau et du TFA 10% aqueux. Après extractions avec Et₂O, la phase aqueuse a été dégazée par bullage d'argon, diluée avec de l'eau, filtrée et purifiée par RP-HPLC sur une colonne C18 vydac 300 Å, 5 µm (215 nm, 20 mL/min, tampon A eau contenant 0,05% TFA, tampon B CH3CN/eau 4/1 contenant 0,05% TFA).

Les fragment 1 et fragment 2 : K1(fragment 1)-MPA et StBu-K1(fragment 2)-SEA sont préparés comme décrit précédemment à l'exemple 2.

### Exemples de préparation des fragments peptidigues de départ

A) La synthèse des fragments peptidiques SEAon est effectuée selon Ollivier N., Dheur J., Mhidia R., Blanpain A., and Melnyk O., Bis(2-sulfanylethyl)amino native peptide ligation., Org. Lett. 12(22), 5238-41 (2010).

A titre d'illustration la préparation des fragments peptidiques
(1a) : H-ILKEPVHGG-N(CH₂CH₂SH)₂,
(1b) : H-ILKEPVHGA-N(CH₂CH₂SH)₂,
(1c) :H-ILKEPVHGV-N(CH₂CH₂SH)₂ et
(1d) :H-ILKEPVHGY-N(CH₂CH₂SH)₂
est effectuée en phase solide

Le fragment peptidique (1a) est obtenu à partir du support amorce préparé avec la glycine.

Le fragment peptidique (1b) est obtenu à partir du support amorce préparé avec l'alanine.

Le fragment peptidique (1c) est obtenu à partir du support amorce préparé avec la valine.

Le fragment peptidique (1d) est obtenu à partir du support amorce préparé avec la tyrosine.

La synthèse des polypeptides peut être résumée comme suit:

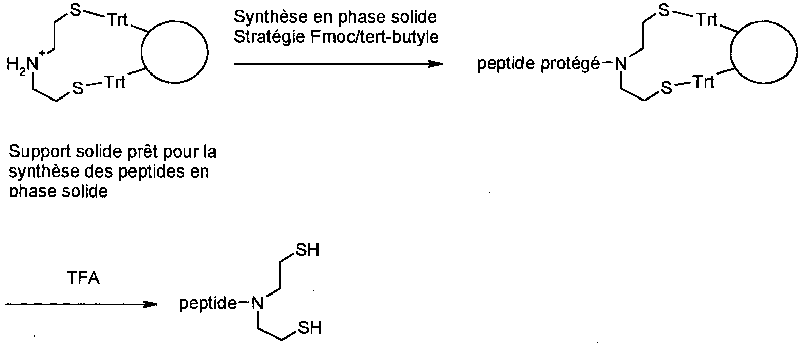

Les fragments peptidiques ainsi obtenus ont la formule générale suivante :

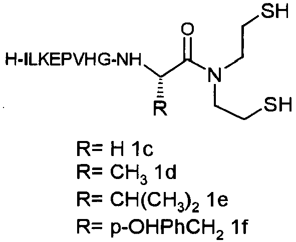

La synthèse des différents polypeptides est effectuée en phase solide en utilisant la stratégie Fmoc/*tert*-butyle sur résines (échelle de 0,1 mmol) sur un synthétiseur de peptide à micro-ondes (CEM µ WAVES, Saclay, France). Le couplage est effectué en utilisant un excès 5 fois molaire de chaque acide aminé, l'activateur HBTU est utilisé avec un excès de 4,5 fois molaire et la base DiEA est utilisée avec un excès 10 fois molaire.

La déprotection finale et le clivage du polypeptide de la résine sont réalisés avec 10 mL d'un mélange TFA/TIS/DMS/H₂O (92,5/2,5/2,5/2,5 en volume) pendant 1 heure. Le polypeptide est ensuite obtenu par précipitation dans 100 mL d'un mélange éther diéthylique/heptane (1/1 en volume), dissout dans H₂O puis lyophilisé.

La pureté de chaque fragment peptidique est déterminée par HPLC (91% pour le fragment peptidique (1a) avec une glycine, 83% pour le fragment peptidique (1b), 80% pour (1c), et 88% pour (1d)). L'analyse MALDI-Tof des fragments peptidiques concorde avec la structure du fragment peptidique attendu (Fragment peptidique (1a) C₄₇H₈₁N₁₃O₁₁S₂ [M+H]⁺ calculé 1068,6 Da, observé 1068,5. Fragment peptidique (1b) C₄₈H₈₃N₁₃O₁₁S₂ [M+H]⁺ calculé 1082,6 Da, observé 1082,4. Fragment peptidique (1c) C₅₀H₈₇N₁₃O₁₁S₂ [M+H]⁺ calculé 1110,6 Da, observé 1110,5). Fragment peptidique 1(d) C₅₄H₈₇N₁₃O₁₂S₂ [M+H]⁺ calculé 1174,6 Da, observé 1174,6).

La purification des polypeptides est réalisée sur colonne C18 nucléosil en acetonitrile-H₂O (80-20) en TFA, avec un gradient de 0 à 30 % en 30 mn pour le fragment peptidique (1a), et un gradient de 0 à 10 % en 5 mn puis 10 à 25% en 25 mn pour les fragment peptidique (1b) et (1c).

La pureté déterminée par HPLC est de 96% pour le fragment peptidique (1a) avec un rendement global de 35%, 97% pour le fragment peptidique (1b) avec un rendement de 31%, et de 99% pour le fragment peptidique (1c) avec un rendement de 27%.

### B) oxydation des fragments peptidiques (1a), (1b), (1c) et (1d) en dithiazépanes

Les fragments peptidiques (1a), (1b), (1c) et (1d) sont tels qu'obtenus précédemment sont oxydés selon le schéma général suivant :

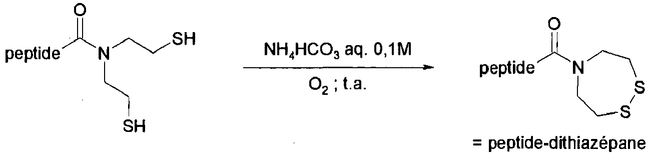

Chaque fragment peptidique est clivé du support solide par l'action d'une solution TFA/DMS/TiS/H₂O (92,5/2,5/2,5/2,5 ; v/v). Le fragment peptidique est alors précipité dans un large volume d'un mélange éther diéthylique/heptane (1/1 ; v/v) et lavé deux fois à l'aide de cette solution. Le fragment peptidique brut lyophilisé après l'étape de coupure est ensuite dissous dans une solution de bicarbonate d'ammonium à 0,1M préalablement dégazée pendant 10 min par bullage d'azote (1 mg/mL).

Le mélange est alors laissé à température ambiante sous agitation forte. L'évolution de la réaction est suivie par spectrométrie de masse MALDI-TOF jusqu'à disparition totale de la forme réduite du polypeptide considéré. Le polypeptide est enfin purifié par RP-HPLC (gradient éluant A (H₂O/0,05% TFA)/éluant B (80% Acétonitrile/20% H₂O/0,05% TFA) : 0 à 10% en 10 min puis 10% à 25 % en 25 min) puis congelé et lyophilisé.

Le tableau ci-dessous résume les résultats obtenus (analyse MALDI-TOF).

| fragment peptidique | m/z [M+H]⁺_{calc.} | M/z [M+H]⁺_{obs.} | Rendement final(%) |
|---|---|---|---|
| (1a) | 1066,6 | 1066,6 | 17 |
| (1b) | 1080,6 | 1080,6 | 13 |
| (1c) | 1108,6 | 1108,6 | 23 |
| (1d) | 1172,6 | 1172,6 | 20 |

## Revendications

1. Un procédé de préparation d'un assemblage peptidique de n fragments et de n-1 acides aminés portant une fonction thiol, représenté par la formule :
A₁-C₁-A₂-C₂-A₃- ... -Cᵢ₋₁-Aᵢ-.... -Cₙ₋₁-Aₙ (I)
dans laquelle A₁, A₂, A₃, ... Aᵢ... , Aₙ sont des fragments peptidiques, C₁, C₂, C₃ ... Cᵢ₋₁ ... Cₙ₋₁ sont des restes d'acides aminés portant une fonction thiol,
n est compris entre 3 et 50, de préférence entre 3 et 20, ou entre 3 et 10, et
i est un nombre entier quelconque compris entre 2 et n,
**caractérisé en ce que** l'on met en oeuvre la préparation d'un peptide-thioester de formule :
A₁-SR (II)
dans laquelle A₁ est un fragment peptidique et SR est le reste d'un thioester alkylé, R pouvant être un radical alkyle éventuellement substitué,
à partir d'un bis(2-sulfanyléthyl)amino peptide : A₁-SEAoff dans laquelle SEAoff est groupement bis(2-sulfanyléthyl)amino cyclique 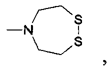 par action d'un thiol R-SH, en présence d'un agent réducteur de disulfures cycliques,
suivie de la condensation sur un fragment peptidique de structure :
H-C₁(SR')-A₂-SEAoff (III)
dans laquelle C₁, A₂ et SEAoff sont définis comme ci-dessus et (SR') représente un reste disulfure sur le thiol de l'amino-acide C₁, en présence d'un thiol aromatique ArSH,
puis transforme le nouveau fragment peptidique obtenu, de structure :
A₁-C₁-A₂-SEAoff (IV)
dans laquelle A₁, C₁, A₂ et SEAoff sont définis comme précédemment, en un peptide-thioester de formule :
A₁-C₁-A₂-SR (II')
dans laquelle A₁, C₁, A₂ et R sont définis comme précédemment, par action d'un thiol R-SH, en présence d'un agent réducteur de disulfures cycliques,
et condense sur un fragment peptidique de structure :
H-C₂(SR')-A₃-SEAoff (III')
dans laquelle SEAoff et R' et C₂ et A₃ sont définis comme ci-dessus, en présence d'un thiol aromatique ArSH, pour donner un fragment peptidique, de structure :
A₁-C₁-A₂-C₂-A₃-SEAoff (IV')
dans laquelle A₁, C₁, A₂, C₂, A₃ et SEAoff sont définis comme précédemment,
et réitère ces 2 opérations jusqu'à n-2 fois, pour obtenir un fragment peptidique de structure :
A₁-C₁-A₂-C₂-A₃- ... -Cᵢ₋₁-Aᵢ-.... -Cₙ₋₂-Aₙ₋₁-SEAoff (IV^{m})
dans laquelle A₁, A₂, A₃, ... Aᵢ ..., Aₙ₋₁, C₁, C₂, C₃ ... Cᵢ₋₁ ... Cₙ₋₂ et SEAoff sont définis comme précédemment, puis
met en oeuvre une réaction de ligation native de ce fragment peptidique (IV^{m}) obtenu, avec un peptide de formule :
H-Cₙ₋₁-Aₙ (Vⁿ)
dans laquelle Cₙ₋₁ et Aₙ sont définis comme précédemment, par réduction en présence d'un agent réducteur de disulfures cycliques, pour donner l'assemblage peptidique multiple de formule générale (I).

2. Un procédé selon la revendication 1, **caractérisé en ce que** l'on prépare un assemblage peptidique de 3 ou 4 fragments, de structures A₁-C₁-A₂-C₂A₃ ou A₁-C₁-A₂-C₂-A₃-C₃-A₄.

3. Un procédé selon la revendication 2, **caractérisé en ce que** l'on prépare un assemblage peptidique de 3 fragments en une réaction « one pot ».

4. Un procédé selon l'une des revendications 1 à 3, **caractérisé en ce que** C₁, C₂, C₃ ... Cᵢ₋₁ ... Cₙ₋₁ représentent des restes Cys.

5. Un procédé selon l'une des revendications 1 à 4, **caractérisé en ce que** l'un ou plusieurs des fragments peptidiques A₂ ... Aᵢ ... Aₙ₋₁ peuvent porter un ou plusieurs amino acides modifiés.

6. Un procédé selon l'une des revendications 1 à 5, **caractérisé en ce que** la condensation du peptide-thioester sur un fragment peptidique de structure (III) s'effectue en présence de l'acide 4-mercaptophénylacétique.

7. Procédé de fabrication d'une composition pharmaceutique **caractérisé en ce qu'**il comprend :
- la fabrication d'un polypeptide ou peptide selon le procédé de fabrication d'un polypeptide de formule (I) selon l'une des revendications 1 à 5, et
- la préparation de compositions pharmaceutiques comprenant un polypeptide ou peptide ainsi préparé, à l'état pur ou en association avec un ou plusieurs adjuvants compatibles et pharmaceutiquement acceptables.

8. Un procédé de fabrication d'un dispositif de diagnostic **caractérisé en ce qu'**il comprend :
- la fabrication d'un polypeptide ou peptide selon le procédé de fabrication d'un polypeptide de formule (I) selon l'une des revendications 1 à 5, et
- la formulation ou la mise en forme de ce polypeptide sous une forme adaptée à une utilisation pour un dispositif de diagnostic.

## Patentansprüche

1. Verfahren zur Herstellung einer Peptid-Assembly aus n Fragmenten und Aminosäuren, die eine Thiol-Funktion tragen, dargestellt durch die Formel:
A₁-C₁-A₂-A₃- .... -Cᵢ₋₁-Aᵢ-.... -Cₙ₋₁-Aₙ (I)
worin A₁, A₂, A₃, ... Aᵢ-..., Aₙ Peptidfragmente sind,
C₁, C₂, C₃ ... Cᵢ₋₁... Cₙ₋₁ Aminosäurereste sind, die eine Thiol-Funktion tragen,
n im Bereich zwischen 3 und 50, bevorzugt zwischen 3 und 20 oder zwischen 3 und 10 liegt, und
i eine beliebige ganze Zahl im Bereich zwischen 2 und n ist,
**dadurch gekennzeichnet, dass** ein Peptid-Thioester mit der folgenden Formel hergestellt wird:
A₁SR (II)
worin A₁ ein Peptidfragment ist und SR der Rest eines alkylierten Thioesters ist, wobei R ein gegebenenfalls substituiertes Alkylradikal sein kann,
aus einem Bis(2-sulfanylethyl)aminopeptid: A₁-SEAoff, worin SEAoff die folgende cyclische Gruppe Bis(2-sulfanylethyl)amino ist : 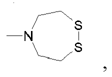 durch Wirkung eines Thiols R-SH, in Gegenwart eines Reduktionsmittels für cyclische Disulfide, gefolgt von der Kondensation an ein Peptidfragment mit der folgenden Struktur:
H-C₁(SR')-A₂-SEAoff (III)
worin C₁, A₂ und SEAoff sind, wie oben definiert, und (SR') für einen Disulfidrest auf dem Thiol der Aminosäure C₁ steht, in Gegenwart eines aromatischen Thiols ArSH,
dann das erhaltene neue Peptidfragment mit der folgenden Struktur:
A₁-C₁-A₂-SEAoff (IV)
worin A_{1'} C₁, A₂ und SEAoff sind, wie vorher definiert, in einen Peptid-Thioester mit der folgenden Formel:
A₁-C₁-A₂-SR (II')
worin A₁, C₁, A₂ und R sind, wie vorher definiert, durch Wirkung eines Thiols R-SH, in Gegenwart eines Reduktionsmittels für cyclische Disulfide transformiert wird,
und auf ein Peptidfragment mit der folgenden Struktur:
H-C₂(SR')-A₃-SEAoff (III')
worin SEAoff und R' und C₂ und A₃ sind, wie oben definiert, in Gegenwart eines aromatischen Thiols ArSH kondensiert wird, um ein Peptidfragment mit der folgenden Struktur zu ergeben:
A₁-C₁-A₂-C₂-A₃-SEAoff (IV')
worin A₁, C₁, A₂, C₂, A₃ und SEAoff sind, wie vorher definiert, und diese 2 Vorgänge bis zu n-2 Mal wiederholt, um ein Peptidfragment mit der folgenden Struktur zu erhalten:
A₁-C₁-A₂-C₂-A₃-... -Cᵢ₋₁-Aᵢ-....-Cₙ₋₂-Aₙ₋₁-SEAoff (IV^{m})
worin A₁, A₂, A₃, ... A₁ ..., Aₙ₋₁, C₁, C₂, C₃ ... Cᵢ₋₁, ... Cₙ₋₂ und SEAoff sind, wie vorher definiert, dann
eine native Ligationsreaktion dieses erhaltenen Peptidfragments (IV^{m}) mit einem Peptid mit der folgenden Formel:
H-Cₙ₋₁-Aₙ (Vⁿ)
worin Cₙ₋₁ und Aₙ sind, wie vorher definiert, durch Reduktion in Gegenwart eines Reduktionsmittels für cyclische Disulfide durchführt, um die Multipeptid-Assembly mit der allgemeinen Formel (I) zu ergeben.

2. Verfahren nach Anspruch 1, **dadurch gekennzeichnet, dass** eine Peptid-Assembly aus 3 oder 4 Fragmenten mit den Strukturen A₁-C₁-A₂-C₂-A₃ oder A₁-C₁-A₂-C₂-A₃-C₃-A₄ hergestellt wird.

3. Verfahren nach Anspruch 2, **dadurch gekennzeichnet, dass** eine Peptid-Assembly aus 3 Fragmenten in einer "Eintopf-Reaktion" hergestellt wird.

4. Verfahren nach einem der Ansprüche 1 bis 3, **dadurch gekennzeichnet, dass** C₁, C₂, C₃ ... Cᵢ₋₁ ... Cₙ₋₁ für Cys-Reste stehen.

5. Verfahren nach einem der Ansprüche 1 bis 4, **dadurch gekennzeichnet, dass** ein oder mehrere der Peptidfragmente A₂ ... Aᵢ ... Aₙ₋₁ eine oder mehrere modifizierte Aminosäuren tragen können.

6. Verfahren nach einem der Ansprüche 1 bis 5, **dadurch gekennzeichnet, dass** die Kondensation des Peptid-Thioesters auf ein Peptidfragment mit der Struktur (III) in Gegenwart von 4-Mercaptophenylessigsäure durchgeführt wird.

7. Verfahren zur Herstellung einer pharmazeutischen Zusammensetzung, **dadurch gekennzeichnet, dass** es Folgendes umfasst:
- die Herstellung eines Polypeptids oder Peptids gemäß dem Verfahren zur Herstellung eines Polypeptids mit der Formel (I) nach einem der Ansprüche 1 bis 5, und
- die Zubereitung von pharmazeutischen Zusammensetzungen, die ein auf diese Weise hergestelltes Polypeptid oder Peptid in reinem Zustand oder in Verbindung mit einem oder mehreren kompatiblen und pharmazeutisch verträglichen Adjuvanzien umfassen.

8. Verfahren zur Herstellung einer Diagnosevorrichtung, **dadurch gekennzeichnet, dass** es Folgendes umfasst:
- die Herstellung eines Polypeptids oder Peptids gemäß dem Verfahren zur Herstellung eines Polypeptids mit der Formel (I) nach einem der Ansprüche 1 bis 5, und
- die Formulierung oder die Formung dieses Polypeptids in eine Form, die für eine Verwendung für eine Diagnosevorrichtung geeignet ist.

## Claims

1. A method for preparing a peptide assembly of n fragments and of n-1 amino acids carrying a thiol function, represented by the formula:
A₁-C₁-A₂-C₂-A₃- ... -Cᵢ₋₁-Aᵢ.... -Cₙ₋₁-Aₙ (I)
wherein A₁, A₂, A₃, ... Aᵢ ... , Aₙ are peptide fragments,
C₁, C₂, C₃ ... Cᵢ₋₁ ... Cₙ₋₁ are amino acid residues carrying a thiol function,
n is comprised between 3 and 50, preferably between 3 and 20, or between 3 and 10 and
i is any integer comprised between 2 and n,
**characterized in that** the preparation is conducted of a peptide-thioester of formula:
A₁-SR (II)
wherein A₁ is a peptide fragment and SR is an alkyl thioester residue, R possibly being an optionally substituted alkyl radical,
from a bis(2-sulfanylethyl)amino peptide: A₁-SEAoff wherein SEAoff is a bis(2-sulfanylethyl)amino cyclic group 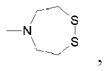 via the action of a thiol R-SH in the presence of a reducing agent of cyclic disulfides, followed by condensation on a peptide fragment of structure:
H-C₁(SR')-A₂-SEAoff (III)
wherein C₁, A₂ and SEAoff are defined as above and (SR') is a disulfide residue on the thiol of the amino acid C₁, in the presence of an aromatic thiol ArSH,
followed by conversion of the new peptide fragment obtained, of structure:
A₁-C₁-A₂-SEAoff (IV)
wherein A₁, C₁, A₂ and SEAoff are as defined previously, to a peptide-thioester of formula:
A₁-C₁-A₂-SR (II')
wherein A₁, C₁, A₂ and R are as defined previously, via action of a thiol R-SH in the presence of a reducing agent of cyclic difsulfides,
and is condensed on a peptide fragment of structure:
H-C₂(SR')-A₃-SEAoff (III')
wherein SEAoff and R' and C₂ and A₃ are defined as above, in the presence of an aromatic thiol ArSH to give a peptide fragment of structure:
A₁-C₁-A₂-C₂-A₃-SEAoff (IV')
wherein A₁, C₁, A₂, C₂, A₃ and SEAoff are as defined previously,
and these 2 operations are reiterated up to n-2 times to obtain a peptide fragment of structure:
A₁-C₁-A₂-C₂-A₃- ... -Cᵢ₋₁- Aᵢ -...-Cₙ₋₂ -Aₙ₋₁-SEAoff (IV^{m})
wherein Ai, A₂, A₃, ... , Aᵢ ..., Aₙ₋₁, C₁, C₂, C₃ ... Cᵢ₋₁ ... Cₙ₋₂ and SEAoff are as defined previously, then,
a native ligation reaction of this obtained peptide fragment (IV^{m}) is conducted with a peptide of formula:
H-Cₙ₋₁-Aₙ (Vⁿ)
wherein Cₙ₋₁ and An are as defined previously, by reduction in the presence of a reducing agent of cyclic disulfides, to give the multiple peptide assembly of general formula (I).

2. A method according to claim 1, **characterized in that** a peptide assembly of 3 or 4 fragments is prepared having structures A₁-C₁-A₂-C₂-A₃ or A₁-C₁-A₂-C₂-A₃-C₃-A₄.

3. A method according to claim 2, **characterized in that** a peptide assembly of 3 fragments is prepared using a one pot reaction.

4. A method according to one of claims 1 to 3, **characterized in that** C₁, C₂, C₃ ... Cᵢ₋₁ ... Cₙ₋₁ represent Cys residues.

5. A method according to one of claims 1 to 4, **characterized in that** one or more peptide fragments A₂ ... Aᵢ ... Aₙ₋₁ may carry one or more modified amino acids.

6. A method according to one of claims 1 to 5, **characterized in that** the condensing of the peptide-thioester on a peptide fragment of structure (III) is conducted in the presence of 4-mercaptophenylacetic acid.

7. A method for manufacturing a pharmaceutical composition **characterized in that** it comprises:
- the manufacture of a polypeptide or peptide according to the method for manufacturing a polypeptide of formula (I) according to one of claims 1 to 5; and
- the preparation of pharmaceutical compositions comprising a polypeptide or peptide thus prepared, in the pure state or in association with one or more compatible adjuvants that are pharmaceutically acceptable.

8. A method for manufacturing a diagnostic device, **characterized in that** it comprises:
- the manufacture of a polypeptide or peptide according to the method for manufacturing a polypeptide of formula (I) according to one of claims 1 to 5; and
- the formulation or forming of this polypeptide in a form adapted for use in a diagnostic device.
